(19) Europäisches Patentamt
European Patent Office
Office européen des brevets

(11) Veröffentlichungsnummer: **0 543 404 A1**

(12) # EUROPÄISCHE PATENTANMELDUNG

(21) Anmeldenummer: **92119818.0**

(51) Int. Cl.$^5$: **C07H 19/073**

(22) Anmeldetag: **20.11.92**

(30) Priorität: **22.11.91 HU 365391**

(43) Veröffentlichungstag der Anmeldung:
**26.05.93 Patentblatt 93/21**

(84) Benannte Vertragsstaaten:
**AT BE CH DE DK ES FR GB GR IE IT LI LU MC NL PT SE**

(71) Anmelder: **Magyar Tudomanyos Akadémia Központi Kémiai Kutato Intézete**
**Pusztaszeri u. 59/67**
**H-1026 Budapest(HU)**

(72) Erfinder: **Ötvös, Laszlo, Dr.**
**Boroka u. 13**
**H-1025 Budapest(HU)**
Erfinder: **Rakoczi, Jozsef, Dr.**
**Fodor u. 18**
**H-1126 Budapest(HU)**
Erfinder: **Szabolcs, Anna, Dr.**
**Martirok utja 29/A**
**H-1024 Budapest(HU)**
Erfinder: **Sagi, Janos, Dr.**
**Lukacs Gy. u. 20**
**H-1038 Budapest(HU)**
Erfinder: **Dékany, Gyula**
**Zsindely u. 11**
**H-1025 Budapest(HU)**
Erfinder: **Szemzo, Attila, Dr.**
**Budaorsi ut 11**
**H-1118 Budapest(HU)**
Erfinder: **Gruber, Lajos, Dr.**
**Cserje u. 18**
**H-1025 Budapest(HU)**
Erfinder: **Nagy, György**
**Hataror u. 28**
**H-1122 Budapest(HU)**
Erfinder: **Nagy, Janos**
**Bürök u. 49**
**H-1124 Budapest(HU)**
Erfinder: **Ivan, Pal**
**Béke u. 18**
**H-1035 Budapest(HU)**
Erfinder: **Tüdos, Helga, Dr.**
**Felho u. 18**
**H-1025 Budapest(HU)**

(74) Vertreter: **von Füner, Alexander, Dr. et al**
**Patentanwälte v. Füner, Ebbinghaus, Finck**
**Mariahilfplatz 2 & 3**
**W-8000 München 90 (DE)**

(54) **Stereoselektives Verfahren zur industriellen Herstellung von 5-Alkyl-2'-desoxy-beta-uridinen.**

(57) DieErfindungbetrifftteinVerfahrenzur stereoselektiven, industriellen Herstellung von 5-Alkyl-2'-desoxy-$\beta$-uridinen der allgemeinen Formel (I)

EP 0 543 404 A1

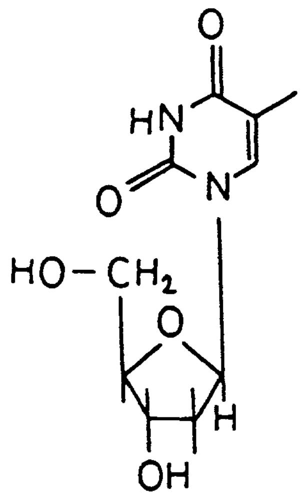

worin R für gerade oder verzweigte Alkylgruppe mit 1 – 8 Kohlenstoffatomen steht. Für das Verfahren ist kennzeichnend, daß
man zu der mit einer minimalen Lösungsmittelmenge bereiteten Lösung von geschützten 5 – Alkyluracylen der allgemeinen Formel (II) – in der allgemeinen Formel (II) ist die Bedeutung von R die gleiche wie oben und X steht für eine Schutzgruppe – unter Kühlung, gegebenenfalls bei Temperaturen unter der Umgebungstempe – ratur und gegebenenfalls unter vermindertem Druck, vorzugsweise geschützte Halogenzucker der

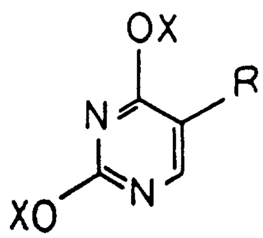

(II)

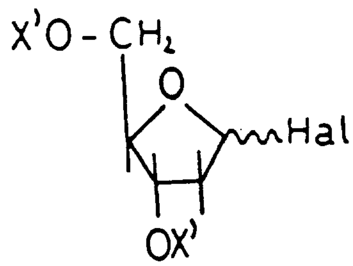

(III)

allgemeinen Formel (III) – in der allgemeinen Formel (III) steht X' für Wasserstoff oder Schutzgruppe und Hal für Halogenatom – gibt und die Schutzgruppen in bekannter Weise, zum Beispiel durch Zemplén – Hydrolyse, entfernt.

Die Erfindung betrifft ein neues, stereoselektives, industrielles Verfahren zur Herstellung von 5 – Alkyl – 2' – desoxy – β – uridinen der allgemeinen Formel (I).

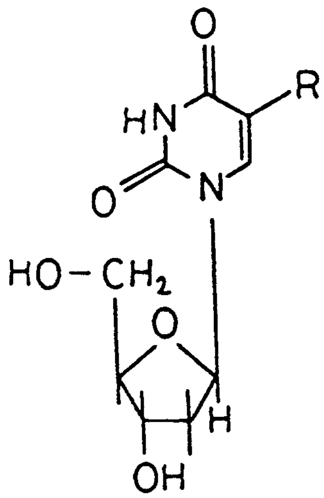

(I)

In der allgemeinen Formel (I) steht R für eine gerade oder verzweigte Alkylgruppe mit 1 – 8 Kohlenstoffato – men.

Von den nicht – natürlichen Mononukleosiden sind in den letzten Jahrzehnten die 5 – substituierten Desoxyuridine stark in den Vordergrund des Interesses von Forschung und Entwicklung getreten, in erster Linie wegen ihrer antiviralen Wirkung. Die Bioaktivität der Verbindungen ist fast ausschließlich den β – Anomeren zuzuordnen. Die β – Anomeren von 5 – Ethyl – und 5 – Isopropyl – 2' – desoxy – uridin sind als gegen Herpes wirksame Verbindungen von bereits eingeführten oder eben in der Phase der Markteinfüh – rung stehenden Präparaten bereits gut bekannt (zum Beispiel die Salbe HEVIZOS$^R$ der Firma BIOGAL in Debrecen, Ungarn, deren Wirkstoff das 5 – Isopropyl – 2' – desoxy – β – uridin ist). Es ist deshalb verständ – lich, daß nach einer rentablen industriellen Synthese gesucht wird. Die Synthese der meisten Nukleoside in großtechnischem Maßstab ist bis in die jüngste Vergangenheit nicht möglich gewesen.

Mit dem erfindungsgemäßen Verfahren wurde eine wirtschaftliche, auch industriell ausführbare Syn – these zur Herstellung von 5 – Alkyl – 2' – desoxy – β – uridinen bereitgestellt.

Aus der Fachliteratur sind zur Herstellung dieser konkreten Verbindung recht wenige Verfahren bekannt. Die für die homologen und analogen Verbindungen (z. B. andere 5 – Alkylverbindungen) bekannten, wesentlich zahlreicheren Verfahren können mit wechselndem Erfolg zur Herstellung der Zielverbindungen herangezogen werden. Das beruht auf dem aus der Literatur bekannten Umstand, daß die in 5 – Stellung befindlichen Substituenten der Uracyle infolge ihrer sterischen und elektrodynamischen Gegebenheiten die Nukleosidbildung in sehr wesentlichem Maße und spezifisch beeinflussen.

Die chemische Synthese ist durch die Kopplung von 2 – Desoxy – D – ribose und 5 – Alkyluracylen realisierbar, jedoch sind in der Fachliteratur auch zahlreiche Beispiele für die Herstellung von 5 – Alkyl – desoxyuridinen aus 5 – substituierten Uridinen beschrieben:

– M. J. Robins et al. stellten 2' – Desoxyuridint aus Uridin in 4 Schritten über das Phenoxythiocarbonyl – derivat durch Reduktion mit Tributylzinn in einer Ausbeute von 68 % her, jedoch ist das Verfahren nur im Labormaßstab geeignet [J. Am. Chem. Soc. 105, 4049 (1983)];
– das Verfahren von Szabolcs et al. (HU 196 824) verfügt über eine größere praktische Bedeutung als das erstgenannte Verfahren, ist jedoch füt eine großtechnische Herstellung ebenfalls nicht geeignet: 5 – substituierte 2' – Chlor – 2' – desoxyuridine werden hergestellt, indem Uridine in Acetonitril bei 100 °C unter Einleiten von Salzsäuregas mit 2 – Acetoxybenzoylchlorid umgesetzt, dann desacetyliert und schließlich das Intermedier in bekannter Weise mit Tributylzinnhydrid reduktiv zu 2' – Desoxyuridin dehalogeniert;

das Verfahren von Ruff et al. (veröffentlichte ungarische Patentanmeldung Nr. 5949/88) ist auch für die industrielle Herstellung geeignet: 5 – Ethyluridin wird mit 2 – Acetoxybenzylchlorid (dieses wird aus Acetylsalicylsäure und Thionylchlorid hergestellt und nicht isoliert) umgesetzt, dann das 5 – Ethyl – 2' – chlor – 3',5' – di – (O – Ac) – 2' – desoxyuridin hergestellt, mit Tributylzinn reduziert und das End – produkt nach der Desacylierung mittels einer neuartigen Aufarbeitung zinnfrei gemacht.

Bei der Ausarbeitung des Verfahrens mußte aus Gründen des Arbeits- und Umweltschutzes davon Abstand genommen werden, bedeutendere Mengen Zinn einzusetzen, deshalb wurde die Methode des Koppelns von 2-Desoxy-D-ribose mit 5-Alkyluracylen gewählt. Bei allen Synthesevarianten dieser Art werden vor der Umsetzung bei beiden Verbindungen die reaktionsfähigen Grppen, die sich an der Umsetzung nicht beteiligen sollen, durch Schutzgruppen geschützt (die 3- und 5-Hydroxylgruppe des Zuckers und die 2- und 4-Hydroxylgruppen des Uracyls), dann wird am glykosidischen 1C-Atom des Zuckers eine sog. aktive (d.h. reaktionsfähige) Gruppe oder ein reaktionsfähiges Atom (im allgemeinen Halogen) eingeführt, und in gewissen Fällen wird auch das 1N-Atom des geschützten Uracyls aktiviert, zum Beispiel mittels Komplexbildung. Das Kondensationsverfahren wird verbreitet angewendet, es entstehen jedoch in jedem Fall $\alpha$- und $\beta$-Anomergemische.

J. J. Fox et al. [JACS 83, 4066 (1961)] koppelten zur Herstellung von 5-Propyl-2'-desoxyuridin das Hg(II)-Salz des N-Acetylderivates von 5-Propyluracyl (5-Propyl-2'-desoxy-uridin) in wasserfreiem Methanol mit dem entsprechend aktivierten Desoxyribofuranosyl-Derivat. In sehr niedriger Umsetzungsrate wurde ein $\alpha,\beta$-Anomergemisch erhalten.

M. Prystas und F. Sorm [Coll. Czech. Chem. Communs. 30, 960-972 (1965)] koppelten 5-Alkyl-2,4-dimethoxypyrimidin-Derivate in Gegenwart von $HgBr_2$ und Molekularsieben mit geschützten Desoxyribofuranosyl-Derivaten. Sie erhielten das Anomergemisch in etwa 20 % Ausbeute.

Die in der französischen Patentschrift Nr. 2 040 177 beschriebene Methode zur Trennung der Anomeren ist sehr umständlich,weil mit extrem verdünnten Lösungen, zum Teil im Milligramm-Maßstab gearbeitet wird.

Viel vorteilhafter ist das Verfahren gemäß der DOS Nr. 2 721 466: die geschützten Reaktionspartner werden in Gegenwart von $HgBr_2$ als Katalysator in Acetonitril miteinander umgesetzt. Es werden 87 % Ausbeute angegeben, durch selektive Kristallisation lassen sich daraus 60 % $\beta$-Anomer und 27 % $\alpha$-Anomer gewinnen.

Wenn das 5-Isopropyl-2'-desoxyuridin nach der Methode von M. Draminski et al. [Polish J. of Chem. 5, 1085-7 (1980)] hergestellt wird, ist der Anteil an $\beta$-Anomer günstiger, das Verfahren ist jedoch für eine industrielle Realisierung nicht geeignet. Die Kondensierung wird in Gegenwart von $SnCl_4$ als Katalysator in Acetonitril vorgenommen, die Gesamtausbeute beträgt 56 %, und das $\beta:\alpha$-Verhältnis ist 10:1.

Gemäß der ungarischen Patentschrift Nr. 171 306 werden die beiden Anomeren auf Grund der Unterschiede in ihrem Lösungs- und Kristallisationsverhalten voneinander getrennt. Das Verfahren beruht im wesentlichen auf einer Verschmelzung der bisher in der Literatur erschienenen Kenntnisse und deren glücklicher Weiterentwicklung. Die geschützten 5-substituierten Uracyle werden in wasserfreiem Acetonitril, in Gegenart von Hg(II)halogeniden und Molekularsieben auf Silikatbasis mit am 1C-Kohlenstoff aktivierten (1-Halogen, im allgemeinen Chlor) Desoxyribofuranosyl-Derivaten kondensiert. Der Vorteil des Verfahrens besteht darin, daß das $\beta$-Anomer selektiv gewonnen werden kann, nachteilig ist jedoch, daß die Ausbeute nur 45-50 % beträgt und die Vorrichtung nicht beliebig groß dimensioniert werden kann; das Einmischen der großen Mengen Molekularsieb und die Extraktion sind problematisch. Die Handhabung der Quecksilbersalze ist gefährlich, und es ist fraglich, ob sie überhaupt in für einen Arzneimittelwirkstoff zureichender Weise entfernbar sind.

Eine Analyse der aus der Literatur bekannten Verfahren kann wie folgt zusammengefaßt werden:

- die Reaktion wird bevorzugt in einem protischen Medium in Gegenwart einer Base oder eines Salzes als Katalysator ausgeführt;
- in jedem Fall erhält man das $\alpha$- und $\beta$-Anomere in unterschiedlichen Verhälnissen enthaltende Gemische. Das 50:50 %-Anomerverhältnis ist häufig, was eindeutig auf einen auf einem Anomergleichgewicht beruhenden Mechanismus hinweist;
- die Reaktionsgeschwindigkeit des mit dem Inversionsmechanismus aus dem $\alpha$-Chlorzucker entstehenden $\beta$-Chlorzuckers ist viermal so groß wie die des $\alpha$-Chlorzuckers; das bedeutet, daß bei einer im Anomergleichgewicht verlaufenden Inversion der Anteil des $\beta$-Nukleosids wesentlich größer sein kann als der entsprechende Anomeranteil im Chlorzucker;
- in Lösung werden die Chlorzucker über einen ionischen Mechanismus, in von der Dielektrizitätskonstante des Lösungsmittels abhängendem Maße anomerisiert. Das Maß der Anomerisation ist in unpolaren Lösungsmitteln, zum Beispiel in Benzol, minimal: 0-15 %. Die Löslichkeit der Chlorzucker in Benzol beträgt jedoch nur 1-1,5 %, deshalb ist Benzol als Reaktionsmedium ungeeignet;
- die Anomerisation kann auch mittels Katalysatoren beeinflußt werden und ist meistens temperaturabhängig [M. P. Kotiek et al.: J. Org. Chem. 34, 3806 (1969)];
- wenn das C1-Carbonium-Kation des Chlorzuckers in dissoziierter Form vorliegt, ist die Bildung des $\alpha$-Nukleosids begünstigt. Den Untersuchungen von Prystas et al. [Coll. Czech. Chem. Communs. 30

3123 (1965)] zufolge wird das Carboniumkation durch die Carbonylgruppe der Acylschutzgruppe in 5C – Stellung in Form eines Ringacetals stabilisiert und blockiert dadurch die β – Position. Ein Teil der Katalysatoren beschleunigt diesen Prozeß. Die komplexbildenden Katalysatoren bilden mit dem 1N – Atom der geschützten Uracylverbindung Komplexe, wodurch im Falle eines ionischen Mechanismus die Entstehung der sterisch günstigeren α – Anomernukleoside begünstigt ist.

Interessanterweise konnte in der Literatur keine Angabe über die Wärmetönung der Kopplungsreaktion gefunden werden. Nach eigenen Experimenten ist die Reaktion schwach exotherm, und die bei der Umsetzung freiwerdende Wärme beeinflußt (insbesondere im Falle größerer Volumina) das Anomerenverhältnis nachteilig, d.h. im Sinne eines Anstieges des α – Nukleosidanteils.

Die Ergebnisse der im Zusammenhang mit einer Optimierung der stereoselektiven Synthese vorgenommenen Versuche sind in der folgenden Tabelle I zusammengefaßt (Arbeitsweise wie in Beispiel 1). Das Reaktionsprodukt wurde in Methanol gekocht, die Lösung abgekühlt und das ausgefallene kristalline Anomer abfiltriert und getrocknet. Das Anomerverhältnis wurde mittels HPLC – Analyse festgestellt.

### Tabelle I

| Wirkung des Lösungsmittels | | | |
|---|---|---|---|
| Lösungsmittel | Gesamtausbeute % | Nukleosidanomerverhältnis % α | β |
| Dichlormethan | 72 | 20 | 80 |
| Dichloräthan | 68 | 30 | 70 |
| Chloroform | 98 | 10 | 90 |
| Benzol | - | - | - |
| Acetonitril | 50 | 80 | 20 |
| **Wirkung der Temperatur** | | | |
| -5 °C | 85 | 10 | 90 |
| 0 °C | 90 | 10 | 90 |
| +5 °C | 98 | 10 | 90 |
| +10 °C | 92 | 10 | 90 |
| +25 °C | 75 | 30 | 70 |
| **Wirkung des Molverhältnisses der Reaktionspartner (auf 10 mMol Chlorzucker)** | | | |
| mMol Silyluracyl | | | |
| 7,5 | 65 | 50 | 50 |
| 10 | 80 | 30 | 70 |
| 11 | 98 | 10 | 90 |

Analytische Überlegungen führten zu der Erkenntnis, daß zur Bildung des β – Mononukleosids die Halogenzuckerkomponente weder im dissoziierten noch im anomerisierten Zustand vorliegen darf, das heißt: statt der in der Literatur vorgeschlagenen Lösungsmittel und statt der in diesen ablaufenden Einphasenreaktion muß zur Erzielung der Stereoselektivität die Reaktion in heterogener oder quasi heterogener Phase geführt werden. Bei Untersuchungen zur Ausführbarkeit dieser Überlegungen wurde überraschenderweise gefunden, daß in fast quantitativer Ausbeute (98 %) ein Anomergemisch des geschützten Nukleosids erhalten werden kann, das zu 92 % das gewünschte β – Anomer enthält, wenn man die kristallinen, zweckmäßig geschützten Halogenzucker und die geschützten 5 – Alkyluracyle in einer minimalen Menge Lösungsmittel suspendiert und so lange rührt, bis die gesamte Menge des festen Halogenzuckers in Reaktion getreten ist.

Gegenstand der Erfindung ist demnach ein industrielles Verfahren zur stereoselektiven Herstellung von 5-Alkyl-2'-desoxy-$\beta$-uridinen der allgemeinen Formel (I) (Formeln siehe Reaktionsschema), worin

R für gerade oder verzweigte Alkylgruppe mit 1-8 Kohlenstoffatomen steht.

Für das erfindungsgemäße Verfahren ist kennzeichnend, daß man zu der mit einer minimalen Lösungsmittelmenge bereiteten Lösung von geschützten 5-Alkyluracylen der allgemeinen Formel (II) - in der allgemeinen Formel (II) ist die Bedeutung von R die gleiche wie oben und X steht für eine Schutzgruppe - unter Kühlung, gegebenenfalls bei Temperaturen unter der Umgebungstemperatur und gegebenenfalls unter vermindertem Druck, vorzugsweise geschützte Halogenzucker der Reaktionsschema

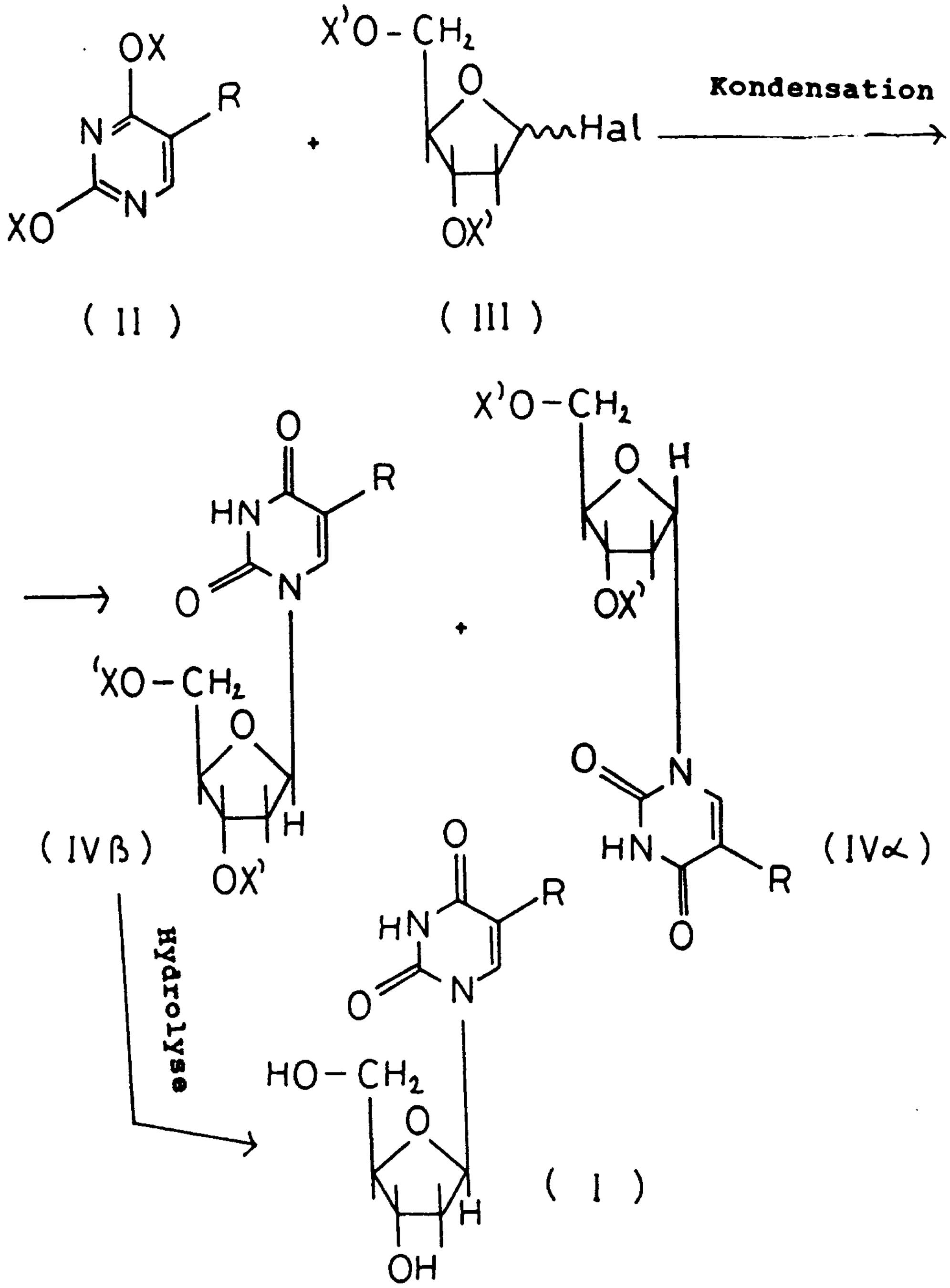


( II ) ( III ) ( IVβ ) ( IVα ) ( I )

allgemeinen Formel (III) - in der allgemeinen Formel (III) steht X' für Wasserstoff oder Schutzgruppe und Hal für Halogenatom - gibt und die Schutzgruppen in bekannter Weise, zum Beispiel durch Zemplén-Hydrolyse, entfernt.

Das Wesen der Erfindung ist demnach die Reaktionsführung in heterogener Phase unter Verwendung einer minimalen Menge Lösungsmittel. Das Lösungsmittel ist vorzugsweise ein chlorierter Kohlenwasserstoff, zum Beispiel Chloroform.

Zu der zum Beispiel mit Chloroform bereiteten, sehr konzentrierten Lösung des geschützten Uracyls, zum Beispiel zu der Lösung von Trimethyl-xylyl-uracyl, wird der zweckmäßig geschützte Halogenzucker in mehreren (zum Beispiel 5 bis 6) Portionen gegeben, so daß immer ein Überschuß an geschütztem Uracyl besteht. Die Umsetzung ist temperaturabhängig und erfordert – im Gegensatz zu der bisherigen Praxis – eine Kühlung von außen. Vorzugsweise wird die Kondensation bei Temperaturen zwischen −5 °C und +10 °C und einem Druck von 2,7-5,4x$10^3$ Pa vorgenommen, damit die Verbindung, die im Laufe der Reaktion aus der Schutzgruppe gebildet wird (zum Beispiel Trimethylsilylchlorid) nicht mit dem nicht umgesetzten Uracyl (zum Beispiel Silyluracyl) einen Komplex bildet, der infolge seiner Flüchtigkeit aus dem Reaktionsgemisch entweicht.

Als Schutzgruppen werden die für derartige Zwecke üblichen verwendet. Besonders bevorzugt sind für X die Trimethylsilylgruppe und für X' die p-Chlorbenzoylgruppe. In der allgemeinen Formel (III) der Halogenzucker steht Hal bevorzugt für Chloratom. In der allgemeinen Formel (I) steht R bevorzugt für eine der niederen Alkylgruppen, insbesondere für Ethyl- oder Isopropylgruppe.

Mit dem erfindungsgemäßen Verfahren steht zur industriellen Herstellung von 5-Alkyl-2'-desoxy-β-uridinen ein wirtschaftliches und sicheres Verfahren zur Verfügung.

Die im Laufe des Verfahrens anfallenden geschützten β-Desoxyuridin-Derivate der allgemeinen Formel (IV), zum Beispiel die 5-Alkyl-3',5'-di-(p-chlorbenzoil)-2'-desoxy-β-uridine, können in bekannter Weise, zum Beispiel mittels Zemplén-Hydrolyse, durch Entfernen der Schutzgruppen zu den Verbindungen der allgemeinen Formel (I) umgesetzt werden.

Das Verfahren wird im folgenden an Hand von Ausführungsbeispielen näher erläutert. Die Herstellung der Ausgangsverbindungen ist an zahlreichen Orten in der Fachliteratur, zum Beispiel in der ungarischen Patentschrift Nr. 196 824, beschrieben.

**Beispiel 1**

**5-Isopropyl-2'-desoxy-β-uridin**

In eine Glasbirne von 50 Liter Volumen, die mit Rührer, Pulverdosiertrichter und Entlüftungsstutzen versehen ist, werden 3310 g (11,09 Mol) 5-Isopropyl-bis(2,4-trimethylsilyl)-uracyl und 12 Liter wasserfreies Chloroform gegeben. Das Gemisch wird unter Rühren auf 0 °C gekühlt. Dann werden 5000 g (10,55 Mol) Chlorzucker, nämlich bis(3,5-p-Chlorbenzoyl)-2-desoxyribofuranosylchlorid, abgewogen. Davon wird 1 kg durch den Pulverdosiertrichter in die Lösung gegeben. Die entstehende Suspension wird bei 0 °C unter vermindertem Druck 30 Minuten lamg gerührt. Alle 30 Minuten wird ein weiteres Kilo Chlorzucker zu der Suspension gegeben, wobei darauf zu achten ist, daß die Temperatur 10-15 °C nicht überschreitet. Nach Zusatz des letzten Kilo Chlorzucker wird der Ansatz so lange gerührt, bis eine homogene Lösung entstanden ist. Die Temperatur steigt in der nächsten halben Stunde auf 20 °C an. Übrigens wird der Verlauf der Reaktion mittels Dünnschichtchromatographie verfolgt. Nach Beendigung der Reaktion wird das Vakuum abgestellt, die Trimethylsilylgruppen werden durch Zusatz von 900 ml technischem Methanol hydrolysiert und das Gemisch wird im Vakuum bis zur Lösungsmittelfreiheit eingedampft. Der Rückstand wird mit 14 Liter technischem Methanol kochend kristallisiert. Das schon in der Wärme ausfallende kristalline Produkt wird abfiltriert und getrocknet. Man erhält 5540 g (90 %) geschütztes β-Anomer (Verbindung der Formel IVβ mit R = Isopropyl und X' = p-Chlorbenzoyl), das auf bekannte Weise mittels der Zemplén-Hydrolyse hydrolysiert wird. Man erhält durch Kristallisation 2450 g 5-Isopropyl-2'-desoxy-β-uridin. Aus der Mutterlauge scheiden sich bei zweitägigem Stehen noch 450 g Produkt ab, das 70-80 % des α-Anomeren enthält. Die Substanz wird mit 98 % Bruttoausbeute abgetrennt.

**Beispiel 2**

**5-Isopropyl-2'-desoxy-β-uridin**

In den gemäß Beispiel 1 ausgerüsteten Behälter werden 6 kg (12,70 Mol) bis(3,5-p-Chlorbenzoyl)-2-desoxy-ribofuranosylchlorid (Chlorzucker) und 3,97 kg (13,3 Mol) bis(2,4-Trimethylsilyl)-5-izopropyluracyl eingewogen. Unter ständigem Rühren und vermindertem Druck werden bei Raumtemperatur 10 Liter, nach 30 Minuten noch einmal 5 Liter wasserfreies Chloroform langsam zugegeben. Die dicke Suspension wird immer dünnflüssiger und ist nach einer Stunde klar. Der Reaktionsverlauf wird dünnschichtchromatographisch verfolgt. Das Produkt gemäß Beispiel 1 – das β-anomere geschützte Desoxyuridin – wird in einer Menge von 6580 g (88 %) erhalten. Aus der methanolischen Mutterlage können 530 g geschütztes 5-Isopropyl-2'-desoxyuridin abgetrennt werden, das zum größten Teil aus α-Anomer besteht.

**Beispiel 3**

**5-Ethyl-3',5'-bis(p-chlorbenzoyl)-2'-desoxy-β-uridin**

In das Reaktionsgefäß gemäß Beispiel 1 werden 6000 g (13,9 Mol) Chlorzucker und 4293 g (15,1 Mol) 5-Ethylsilyluracyl sowie 26 Liter Chloroform gegeben. Letzteres enthält weniger als 0,5 Masse% Wasser. Das Gemisch wird unter Kühlung bei 10-15 °C gerührt. Aus der Suspension bildet sich innerhalb von 2,5 Stunden eine homogene Lösung. Unter ständigem Rühren läßt man 26 Liter Methanol einfließen, wobei sich das Produkt in fester Form ausscheidet. Es wird abfiltriert und getrocknet. Man erhält 6820 g 5-Ethyl-3',5'-bis(p-chlorbenzhoyl)-2'-desoxyuridin, das zu 95-96 % aus dem β-Anomer besteht. Die Schutzgruppen werden wie bereits beschrieben unter Beibehaltung der Anomerie entfernt.

**Patentansprüche**

1. Verfahren zur stereoselektiven, industriellen Herstellung von 5-Alkyl-2'-desoxy-β-uridinen der allgemeinen Formel (I)

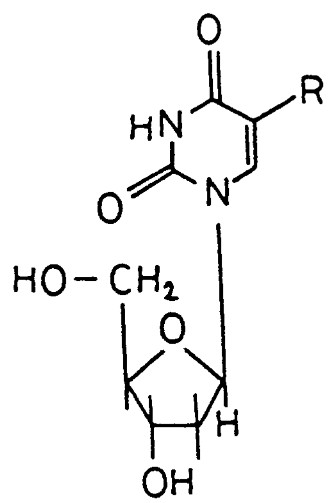

worin

R für gerade oder verzweigte Alkylgruppe mit 1-8 Kohlenstoffatomen steht,

dadurch gekennzeichnet, daß

man zu der mit einer minimalen Lösungsmittelmenge bereiteten Lösung von geschützten 5-Alkyluracylen der allgemeinen Formel (II) – in der allgemeinen Formel (II) ist die Bedeutung von R die gleiche wie oben und X steht für eine Schutzgruppe – unter Kühlung, gegebenenfalls bei Temperaturen unter der Umgebungstemperatur und gegebenenfalls unter vermindertem Druck, vorzugsweise geschützte Halogenzucker der

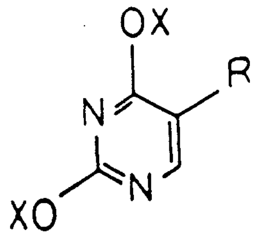

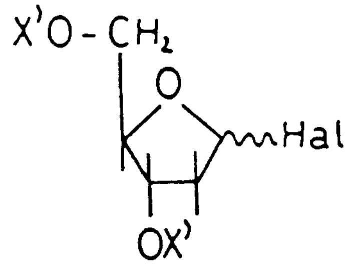

(II) (III)

allgemeinen Formel (III) – in der allgemeinen Formel (III) steht X' für Wasserstoff oder Schutzgruppe und Hal für Halogenatom – gibt und die Schutzgruppen in bekannter Weise, zum Beispiel durch Zemplén – Hydrolyse, entfernt.

**2.** Verfahren nach Anspruch 1, dadurch gekennzeichnet, daß man von einem 5 – Alkyl – uracyl – Derivat ausgeht, in dem R für Ethylgruppe steht.

**3.** Verfahren nach Anspruch 1, dadurch gekennzeichnet, daß man von einem 5 – Alkyl – uracyl – Derivat ausgeht, in dem R für Isopropylgruppe steht.

**4.** Verfahren nach einem der Ansprüche 1 – 3, dadurch gekennzeichnet, daß man von einem 5 – Alkyl – uracyl – Derivat ausgeht, in dem X für Trimethylsilyl steht.

**5.** Verfahren nach einem der Ansprüche 1 – 4, dadurch gekennzeichnet, daß man von einem geschützten Halogenzucker ausgeht, in dem X' für p – Chlorbenzoyl steht,

**6.** Verfahren nach einem der Ansprüche 1 – 5, dadurch gekennzeichnet, daß man von einem Halogen – zucker ausgeht, in dem Hal für Chlor steht.

**7.** Verfahren nach einem der Ansprüche 1 – 6, dadurch gekennzeichnet, daß man die Halogenzucker der allgemeinen Formel (III) in mehreren, zweckmäßig 5 – 6, einzelnen Portionen zu der Lösung der 5 – Alkyluracyle der allgemeinen Formel (II) gibt.

**8.** Verfahren nach einem der Ansprüche 1 – 7, dadurch gekennzeichnet, daß man die Umsetzung bei – 5 °C bis + 10 °C vornimmt.

**9.** Verfahren nach einem der Ansprüche 1 – 8, dadurch gekennzeichnet, daß man die Umsetzung bei einem Druck von 2,7 – 5,4 x $10^3$ Pa vornimmt.

**10.** Verfahren nach einem der Ansprüche 1 – 9, dadurch gekennzeichnet, daß man als Lösungsmittel halogenierte IKohlenwasserstoffe einsetzt.

**11.** Verfahren nach einem der Ansprüche 1 – 10, dadurch gekennzeichnet, daß man als Lösungsmittel Chloroform einsetzt.

Europäisches Patentamt

# EUROPÄISCHER RECHERCHENBERICHT

Nummer der Anmeldung

EP 92 11 9818

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X<br>Y | EP-A-0 300 765 (ETHYL CORPORATION)<br>* das ganze Dokument * | 1-11<br>1-11 | C07H19/073 |
| X<br>Y | DE-A-1 620 185 (ROBUGEN GMBH)<br>* das ganze Dokument * | 1-11<br>1-11 | |
| Y | DE-A-2 721 466 (ROBUGEN GMBH)<br>* Ansprüche 1,2 * | 1-11 | |
| Y | EP-A-0 208 550 (THE WELLCOME FOUNDATION LTD.)<br>* Seite 8, Zeile 20 - Seite 12, Zeile 3 * | 1-11 | |
| Y | US-A-4 468 384 (T.J.BARDOS ET AL.)<br>* Spalte 3, Zeile 29 - Spalte 4, Zeile 8 * | 1-11 | |
| Y | US-A-5 028 596 (D.J.M.PURIFOY ET AL.)<br>* Spalte 6, Zeile 55 - Spalte 8, Zeile 40 * | 1-11 | |
| Y | EP-A-0 010 205 (SLOAN-KETTERING INSTITUTE FOR CANCER RESEARCH)<br>* Seite 6, Zeile 10 - Seite 15, Zeile 15 * | 1-11 | RECHERCHIERTE SACHGEBIETE (Int. Cl.5)<br>C07H |
| Y | EP-A-0 417 560 (THE WELLCOME FOUNDATION LTD.)<br>* Seite 7, Zeile 43 - Seite 10, Zeile 4 * | 1-11 | |
| Y | EP-A-0 389 110 (YUKEI GOSEI KOGYO GO., LTD.)<br>* das ganze Dokument * | 1-11 | |

-----

Der vorliegende Recherchenbericht wurde für alle Patentansprüche erstellt

| Recherchenort | Abschlußdatum der Recherche | Prüfer |
|---|---|---|
| DEN HAAG | 29 JANUAR 1993 | SCOTT J.R. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument